# EUROPEAN PATENT APPLICATION

(11) **EP 4 026 544 A1**
(43) Date of publication of application: **13.07.2022**
(21) Application number: 20861197.0
(22) Date of filing: 30.07.2020
(51) Int. Cl.: A61K 31/165, A61K 31/4164, A61K 39/395, A61P 35/00, C07K 16/28, A61K 39/00

(54) **PHARMACEUTICAL COMPOSITION COMPRISING HDAC INHIBITOR AND ANTI-PD1 ANTIBODY OR ANTI PD-L1 ANTIBODY**

(30) Priority: 04.09.2019 KR 20190109256
(71) Applicant: Crystalgenomics, Inc., Seongnam-si, Gyeonggi-do 13488 (KR)
(72) Inventor: KIM, Young-Dae, Gwangju-si Gyeonggi-do 12771 (KR); CHO, Joong Myung, Seoul 05553 (KR)
(74) Representative: Schön, Christoph
(86) International application number: PCT/KR2020/010059
(87) International publication number: WO 2021/045392

(57) **Abstract**

The present invention relates to a method for killing cancer cells by using a combination of an HDAC inhibitor and an anti-PD1 antibody or an anti PD-L1 antibody. The present invention can have an effective influence on: extending the survival of animals with cancer, tumors, tumor-related disorders and neoplastic diseases; inhibiting the growth of proliferative cells associated with neoplasms; or neoplastic degeneration.

## Description

### Technical Field

The present invention relates to a method for inhibiting the growth of cancer cells and killing cancer cells by using a combination of an HDAC inhibitor and an anti-PD-1 antibody or an anti-PD-L1 antibody.

This application claims priority over the use of alkylcarbamoyl naphthalenyloxy octenoyl hydroxyamide, a derivative thereof or a salt thereof in combination with an anti-PD-1 antibody in carcinoma, since the applicant has demonstrated that the specific immunological activity of an epigenetic target inhibitor, alkylcarbamoyl naphthalenyloxy octenoyl hydroxyamide, a derivative thereof, or a salt thereof has synergy in the anti-cancer effect of the anti-PD-1 antibody in Korean Patent No. 1978364, registered on May 8, 2019, entitled "PHARMACEUTICALLY ACCEPTABLE SALT OF ALKYLCARBAMOYL NAPHTHALENYLOXY OCTENOYLHYDROXYAMIDE OR OF DERIVATIVE THEREOF AND METHOD FOR PREPARING SAME."

In addition, this application claims the benefit of priority from Korean Patent Application No. 2019-0109256, filed on September 4, 2019, all contents of which are incorporated herein as a part of this specification.

### Background Art

Histones are basic proteins that bind to DNA in the nucleus of eukaryotic cells and undergo reversible acetylation of the amino groups of lysine residues at specific positions in each molecule of histones. The acetylation of histones is related to the formation of higher structures of the chromatin or the cell division cycle, and thus it is involved in the regulation of the expression of genetic information and it is stably regulated by histone acetyltransferases (HATs) and histone deacetylases (HDACs). It is known that these enzymes neutralize positive charges of lysine residues (four residues for H4) at the amino terminus of histones by acetylation to induce transcriptional activity, or deacetylate them to give charge again to inhibit transcription, thereby inducing equilibrium of acetylation levels of histones and regulating gene expression in the phase of transcription.

HDAC has recently been found to play a role in promoting cell proliferation by being highly expressed in poor environmental conditions such as hypoxia, low glucose and cell carcinogenesis to inhibit expression of cell proliferation inhibitors. Therefore, it has been recognized as an important factor in regulating carcinogenicity and differentiation of cells. In other words, if high acetylation of chromatin inhibits cell proliferation and promotes differentiation, HDAC plays a crucial role in inducing cell proliferation through deacetylation of histones. This is supported by the fact that treatment of HDAC inhibitors results in inhibition of cell proliferation and angiogenesis.

Meanwhile, PD-1 is known to play important roles in regulating immune responses and maintaining peripheral tolerance. PD-1 is moderately expressed on naive T cells, B-cells, and NKT cells, and has expression regulated by T/B cell receptor signaling on lymphocytes, monocytes and bone marrow cells.

PD-1 has two known ligands, PD-L1 (B7-H1) and PD-L2 (B7-DC), which are expressed on cancer cells in a variety of tissues. Specifically, in cells of various cancers, such as ovarian cancer, kidney cancer, colorectal cancer, pancreatic cancer, liver cancer and melanoma, PD-L1 expression correlates with poor prognosis and reduces overall survival, regardless of subsequent treatment.

Therefore, it is understood that interaction of PD-L1 on tumor cells with PD-1 on T cells reduces T cell activation and decreases immune surveillance functions, thus contributing to an impaired immune response against tumors.

It is predicted that the efficacy of these antibodies can be further enhanced by using in combination with radiotherapy, surgery, chemotherapy, targeted therapy, signaling pathway inhibitors, immune enhancers, and the like.

Therefore, in order to effectively apply to the growth reduction and necrosis of cancer cells, there is a need for a study on a method for treating a combination of an appropriate inhibitor and an antibody as described above.

### Detailed Description of the Invention

### Technical Problem

In order to solve the above problems, the present invention provides a method for killing cancer cells by using an HDAC inhibitor in combination with an anti-PD-1 antibody or an anti-PD-L1 antibody.

### Solution to Problem

The present invention provides a pharmaceutical composition comprising:
alkylcarbamoyl naphthalenyloxy octenoyl hydroxyamide, a derivative thereof or a salt thereof; and
an anti-PD-1 antibody or an anti-PD-L1 antibody.

According to one embodiment, the weight ratio of the alkylcarbamoyl naphthalenyloxy octenoyl hydroxyamide, a derivative thereof or a salt thereof to the anti-PD-1 antibody or the anti-PD-L1 antibody may be 1:0.1 to 1:15.

According to one embodiment, the anti-PD-1 antibody or the anti-PD-L1 antibody may be administered at a dose of 1 to 200 mg/kg.

According to one embodiment, the alkylcarbamoyl naphthalenyloxy octenoyl hydroxyamide, a derivative thereof or a salt thereof may be administered at a dose of 10 to 500 mg/kg.

According to one embodiment, the anti-PD-1 antibody may comprise one or more selected from the group consisting of pembrolizumab, nivolumab, camrelizumab, cemiplimab, sintilimab and toripalimab.

According to one embodiment, the anti-PD-L1 antibody may comprise one or more selected from the group consisting of atezolizumab, avelumab and durvalumab.

According to one embodiment, the alkylcarbamoyl naphthalenyloxy octenoyl hydroxyamide or a derivative thereof may be one or more selected from the group consisting of following compounds:
1) (E)-N1-(3-(1H-imidazol-1-yl)propyl)-N8-hydroxy-2-((naphthalen-1-yloxy)methyl)octenediam ide,
2) (E)-N8-hydroxy-N1-(4-hydroxyphenethyl)-2-((naphthalen-1-yloxy)methyl)-2-octenediamide,
3) (E)-N1-(3-(dimethylamino)-2,2-dimethylpropyl)-N8-hydroxy-2-((naphthalen-1-yloxy)methyl)octenediamide,
4) (E)-N1-(2-(diisopropylamino)ethyl)-N8-hydroxy-2-((naphthalen-1-yloxy)methyl)octenediam ide,
5) (E)-N8-hydroxy-N1-(1-methoxypropan-2-yl)-2-((naphthalen-1-yloxy)methyl)-2-octenediamide,
6) (E)-N8-hydroxy-N1-(4-methoxybenzyl)-2-((naphthalen-1-yloxy)methyl)-2-octenediamide,
7) (E)-N1-(4-fluorophenethyl)-N8-hydroxy-2-((naphthalen-1-yloxy)methyl)-2-octenediamide,
8) (E)-N8-hydroxy-2-((naphthalen-1-yloxy)methyl)-N1-(tetrahydrofuran-2-yl)methyl)-2-octenediamide,
9) (E)-N1-(2-cyclohexenylethyl)-N8-hydroxy-2-((naphthalen-1-yloxy)methyl)-2-octenediamide,
10) (E)-N8-hydroxy-2-((naphthalen-1-yloxy)methyl)-N1-(3-(2-oxopyrrolidin-1-yl)propyl)-2-octenediamide,
11) (E)-N1-(furan-2-ylmethyl)-N8-hydroxy-2-((naphthalen-1-yloxy)methyl)-2-octenediamide,
12) (E)-N1-(4-(dimethylamino)benzyl)-N8-hydroxy-2-((naphthalen-1-yloxy)methyl)-2-octenediamide,
13) (E)-N8-hydroxy-N1-(2-methoxyethyl)-2-((naphthalen-1-yloxy)methyl)-2-octenediamide,
14) (E)-N1-cyclohexyl-N8-hydroxy-2-((naphthalen-1-yloxy)methyl)-2-octenediamide,
15) (E)-N8-hydroxy-2-((naphthalen-1-yloxy)methyl)-N1-(thiophen-2-ylmethyl)-2-octenediamide,
16) (E)-N8-hydroxy-N1-(4-methoxyphenethyl)-2-((naphthalen-1-yloxy)methyl)-2-octenediamide,
17) (E)-N8-hydroxy-2-((naphthalen-1-yloxy)methyl)-N1-(4-(trifluoromethoxy)benzyl)-2-octenediamide,
18) (E)-N1-(1-(cyclohexylmethyl)pyrrolidin-3-yl)-N8-hydroxy-2-((naphthalen-1-yloxy)methyl)-2-octenediamide,
19) (E)-N1-(1-cyclopentylpiperidin-4-yl)-N8-hydroxy-2-((naphthalen-1-yloxy)methyl)-2-octenediamide,
20) (E)-N1-(1-benzylpyrrolidin-3-yl)-N8-hydroxy-2-((naphthalen-1-yloxy)methyl)-2-octenediamide,
21) (E)-N8-hydroxy-N1-(1-isopropylpyrrolidin-3-yl)-2-((naphthalen-1-yloxy)methyl)-2-octenediamide,
22) (E)-N1-(1-(cyclohexanecarbonyl)pyrrolidin-3-yl)-N8-hydroxy-2-((naphthalen-1-yloxy)methyl)-2-octenediamide,
23) (E)-3-(8-(hydroxyamino)-2-((naphthalen-1-yloxy)methyl)-8-oxo-2-octenamido)pyrrolidine-1-carboxylic acid t-butyl ester,
24) (E)-N8-hydroxy-2-((naphthalen-1-yloxy)methyl)-N1-(pyrrolidin-3-yl)-2-octenediamide,
25) (E)-N1-(1-cyclohexylpyrrolidin-3-yl)-N8-hydroxy-2-((naphthalen-2-yloxy)methyl)-2-octenediamide,
26) (E)-N1-(1-cyclopropylpyrrolidin-3-yl)-N8-hydroxy-2-((naphthalen-1-yloxy)methyl)-2-octenediamide,
27) (E)-N1-(1-cyclopropylpiperidin-4-yl)-N8-hydroxy-2-((naphthalen-1-yloxy)methyl)-2-octenediamide,
28) (E)-N1-(1-ethylpiperidin-4-yl)-N8-hydroxy-2-((naphthalen-1-yloxy)methyl)-2-octenediamide,
29) (E)-N1-(1-ethylpyrrolidin-3-yl)-N8-hydroxy-2-((naphthalen-1-yloxy)methyl)-2-octenediamide,
30) (E)-N8-hydroxy-N1-(2-(1-methylpyrrolidin-2-yl)ethyl)-2-((naphthalen-1-yloxy)methyl)-2-octenediamide,
31) (E)-N8-hydroxy-N1-(1-isopropylpiperidin-4-yl)-2-((naphthalen-1-yloxy)methyl)-2-octenediamide, and
32) (E)-N1-(3-(dimethylamino)propyl)-N8-hydroxy-2-((naphthalen-1-yloxy)methyl)-2-octenediamide.

According to one embodiment, the salt of alkylcarbamoyl naphthalenyloxy octenoyl hydroxyamide may be (E)-N1-(3-(dimethylamino)propyl)-N8-hydroxy-2-((naphthalene-1-yloxy)methyl)-2-octenediamide phosphate.

According to one embodiment, the alkylcarbamoyl naphthalenyloxy octenoyl hydroxyamide, a derivative there of or a salt thereof; and
the anti-PD-1 antibody or the anti-PD-L1 antibody may be administered simultaneously as a single preparation, or may be administered simultaneously or sequentially or in reverse sequence as a separate preparation.

According to one embodiment, the alkylcarbamoyl naphthalenyloxy octenoyl hydroxyamide, a derivative thereof or a salt thereof; and
the anti-PD-1 antibody or the anti-PD-L1 antibody may be prepared as a single preparation.

According to one embodiment, the alkylcarbamoyl naphthalenyloxy octenoyl hydroxyamide, a derivative thereof or a salt thereof; and
the anti-PD-1 antibody or the anti-PD-L1 antibody may be prepared in a separate composition, respectively.

According to one embodiment, the alkylcarbamoyl naphthalenyloxy octenoyl hydroxyamide, a derivative thereof or a salt thereof; and
the anti-PD-1 antibody or the anti-PD-L1 antibody may be administered at a dose less than or equal to a therapeutically effective dose, respectively.

According to one embodiment, 1) the alkylcarbamoyl naphthalenyloxy octenoyl hydroxyamide, a derivative thereof or a salt thereof is administered prior to administration of the anti-PD-1 antibody or the anti-PD-L1 antibody, or
2) the anti-PD-1 antibody or the anti-PD-L1 antibody may be administered prior to administration of the alkylcarbamoyl naphthalenyloxy octenoyl hydroxyamide, a derivative thereof or a salt thereof.

According to one embodiment, the alkylcarbamoyl naphthalenyloxy octenoyl hydroxyamide, a derivative thereof or a salt thereof, and the anti-PD-1 antibody or the anti-PD-L1 antibody may be formulated as a separate preparation such that the alkylcarbamoyl naphthalenyloxy octenoyl hydroxyamide, a derivative thereof or a salt thereof is administered intravenously or orally to an animal and the anti-PD-1 antibody or the anti-PD-L1 antibody is administered intravenously to the animal.

According to one embodiment, the composition may be synergistic in inhibiting the growth of or killing cancer cells.

According to one embodiment, the cancer cells may be characterized by the expression of PD-L1, and the cancer cells include liver cancer cells by hepatitis A virus, liver cancer cells by hepatitis B virus, liver cancer cells by hepatitis C virus, non-viral-related liver cancer cells, metastatic liver cancer cells, colon cancer cells, pancreatic cancer cells, blood cancer cells, melanoma cells or lung cancer cells.

According to one embodiment, the cancer cells may be screened by a method comprising measuring the expression of PD-L1 in the tumor tissue.

According to one embodiment, PD-L1 tumor proportion score (TPS) on the cancer cells may be 1% or more.

The specific details of other embodiments of the present invention are included in the detailed description below.

### Effect of the Invention

The method for killing cancer cells by using an HDAC inhibitor in combination with an anti-PD-1 antibody or an anti-PD-L1 antibody according to the present invention can effectively kill cancer cells.

Cancers, tumors, tumor-related disorders and neoplastic diseases are usually life-threatening conditions which are characterized by rapidly-proliferating cell growth. The present invention can have an effective influence on: extending the survival of animals with the above diseases or disorders; inhibiting the growth of proliferative cells associated with neoplasms; or neoplastic degeneration.

### Brief Description of Drawings

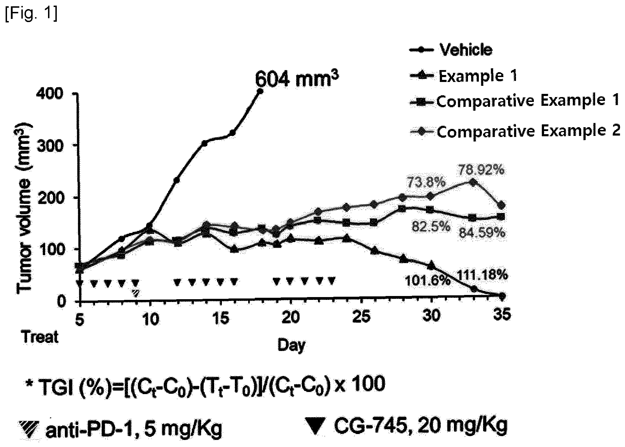

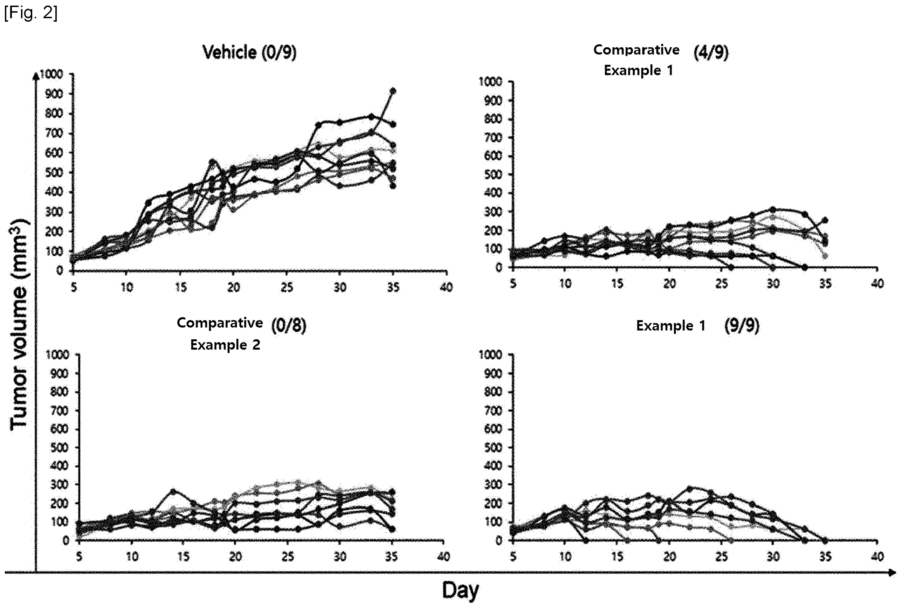

Figs. 1 and 2 are graphs showing the tumor growth inhibition rate in a mouse model.

### Best Mode for Carrying out the Invention

Since various modifications and variations can be made in the present invention, particular embodiments are illustrated in the drawings and will be described in detail in the detailed description. It should be understood, however, that the invention is not intended to be limited to the particular embodiments, but includes all modifications, equivalents, and alternatives falling within the spirit and scope of the invention. In the following description of the present invention, detailed description of known functions will be omitted if it is determined that it may obscure the gist of the present invention.

Hereinafter, a method for killing cancer cells by using a combination of an HDAC inhibitor and an anti-PD-1 antibody or an anti-PD-L1 antibody according to embodiments of the present invention will be described in more detail.

As used herein, the term "abnormal cell growth" refers to cell growth independent of normal regulatory mechanisms, including the abnormal growth of normal cells and the growth of abnormal cells, such as loss of contact inhibition. That is, the mechanism is lost that is involved in the inhibition of growth and division of cells when cells contact with neighboring cells or tissues as they grow.

As used therein, the term "neoplasia" refers to an abnormal, unregulated and disorganized proliferation of cells that is distinguished from normal cells by autonomous growth and somatic mutations. As neoplastic cells grow and divide, they pass on their genetic mutations and proliferative characteristics to progeny cells. A neoplasm, or tumor, is an accumulation of neoplastic cells. In some embodiments, the neoplasm can be benign or malignant.

As used herein, the term "metastasis" refers to the dissemination of tumor cells via lymphatics or blood vessels. Metastasis also refers to the migration of tumor cells by direct extension through serous cavities, or subarachnoid or other spaces. Through the process of metastasis, tumor cell migration to other areas of the body establishes neoplasms in areas away from the site of initial appearance.

As used herein, the term "subject" refers to animals including, but not limited to, primates (e.g., humans), cows, sheep, goats, horses, dogs, cats, rabbits, rats, or mice. The terms "subject" and "patient" are used interchangeably herein with reference to, for example, a mammalian subject, such as a human subject.

As used herein, the terms "treat", "treating" and "treatment" are meant to include alleviating or abrogating a disorder, disease, or condition; or one or more of the symptoms associated with the disorder, disease, or condition; or alleviating or eradicating the cause(s) of the disorder, disease, or condition itself.

As used herein, the term "therapeutically effective amount" refers to the amount of a compound that, when administered, is sufficient to prevent development of, or alleviate to some extent, one or more of the symptoms of the disorder, disease, or condition being treated. The term "therapeutically effective amount" also refers to the amount of a compound that is sufficient to elicit the biological or medical response of a cell, tissue, system, animal, or human that is being sought by a researcher, veterinarian, medical doctor, or clinician.

As used herein, the term "pharmaceutical composition" can be used interchangeably with "pharmacological composition" and "pharmaceutically acceptable composition" and means compositions which can be a relatively non-toxic to a subject to be administered and have harmless effective action. In addition, it may mean any organic or inorganic compound formulation in that side effects resulting from the composition do not impair the efficacy of the drug, that does not cause serious irritation to the subject to which the compound is administered, and does not impair the biological activity and properties of the compound.

As used herein, the term "pharmaceutically acceptable carrier," "pharmaceutically acceptable excipient," "physiologically acceptable carrier," or "physiologically acceptable excipient" refers to a pharmaceutically acceptable material, composition, or vehicle, such as a liquid or solid filler, diluent, excipient, solvent, or encapsulating material. Each component must be "pharmaceutically acceptable" in the sense of being compatible with the other ingredients of a pharmaceutical formulation. It must also be suitable for use in contact with the tissue or organ of humans and animals without excessive toxicity, irritation, allergic response, immunogenicity, or other problems or complications, commensurate with a reasonable benefit/risk ratio. See, Remington: The Science and Practice of Pharmacy, 21st Edition; Lippincott Williams & Wilkins: Philadelphia, PA, 2005; Handbook of Pharmaceutical Excipients, 5th Edition; Rowe et al, Eds., The Pharmaceutical Press and the American Pharmaceutical Association: 2005; and Handbook of 'Pharmaceutical Additives, 3rd Edition; Ash and Ash Eds., Gower Publishing Company: 2007; Pharmaceutical Preformulation and Formulation, Gibson Ed., CRC Press LLC: Boca Raton, FL, 2004.

As used herein, the term "pharmaceutical composition" refers to a mixture of a compound disclosed herein and other chemical ingredients, such as pharmaceutically acceptable diluents, carriers, and the like. The compound can be easily administered to an organism via the pharmaceutical composition.

In the present invention, each constituent drug of the pharmaceutical composition may be present as a separate preparation or a single preparation, and the constituent drugs may be administered simultaneously, sequentially or in reverse sequence. In addition, in the present invention, the pharmaceutically effective amount, administration time, administration interval, administration route, treatment period, etc. of each constituent drug of the pharmaceutical composition may be the same or different from each other.

As used herein, the term "subject to be administered" can be used interchangeably with "individual to be administered" and "organism to be administered" and refers to all animals, including humans, that have been infected or can be injected with bacteria or resistant strains.

As used herein, the term "death" includes both of meanings of necrosis and apoptosis.

As discussed herein, "angiogenesis" is important in tumorigenesis and metastasis of the tumor. Angiogenic factors have been found to be associated with several solid tumors, such as rhabdomyosarcoma, retinoblastoma, Ewing's sarcoma, neuroblastoma, and osteosarcoma. Tumors cannot expand without a blood supply that provides nutrients and provides cellular waste. Tumors for which angiogenesis is important include solid tumors such as renal cell carcinoma, hepatocellular carcinoma and benign tumors such as acoustic neuroma and neurofibromatosis. Angiogenesis is associated with blood-borne tumors such as leukemia. It is believed that angiogenesis may play a role in the abnormalities in the bone marrow that give rise to leukemias. Prevention of angiogenesis can stop the growth of cancerous tumors and consequently damage to the subject due to the presence of the tumor.

The present invention provides a pharmaceutical composition and a method for inhibiting growth of or killing cancer cells by using a combination of an HDAC inhibitor and an anti-PD-1 antibody or an anti-PD-L1 antibody.

The HDACs are a family including at least eighteen enzymes, grouped in three classes (Class I, II and III). Class I HDACs include, but are not limited to, HADCs 1, 2, 3, and 8. Class I HDACs can be found in the nucleus and are believed to be involved with transcriptional control repressors. Class II HDACs include, but are not limited to, HDACS 4, 5, 6, 7, and 9 and can be found in both the cytoplasm as well as the nucleus. Class III HDACs are believed to be NAD dependent proteins and include, but are not limited to, members of the sirtuin family of proteins. Non-limiting examples of sirtuin proteins include SIRT1-7. As used herein, the term "selective HDAC" refers to an HDAC inhibitor that does not interact with all the three HDAC classes.

HDAC inhibitors are a class of therapeutic agents that promote differentiation and apoptosis in hematologic and solid malignancies through chromatin remodeling and gene expression regulation. Several HDAC inhibitors have been identified including benzamides (entinostat), short-chain fatty acids (i.e., sodium phenylbutyrate); hydroxamic acids (i.e., vorinostat and trichostatin A); cyclic tetrapeptides containing a 2-amino-8-oxo-9,10-epoxy-decanoyl moiety (i.e., trapoxin A) and cyclic peptides without the 2-amino-8-oxo-9,10-epoxy-decanoyl moiety (i.e., FK228).

HDAC inhibitors can be classified broadly into pan-HDAC inhibitors and selective HDAC inhibitors. Although there is a large structural diversity of known HDAC inhibitors, they share common features: a part that interacts with the enzyme active site and a side-chain that sits inside the channel leading to the active site. This can be seen with the hydroxamates such as SAHA (suberoylanilide hydroxamic acid), where the hydroxamate group is believed to interact with the active site. In the case of the depsipeptides, it is believed that an intracellular reduction of the disulfide bond creates a free thiol group (which interacts with the active site) attached to a 4-carbon alkenyl chain.

A difference between the HDAC inhibitors is in the way that they interact with the rim of the HDAC channel, which is at the opposite end of the channel to the active site. This interaction between the HDAC inhibitor and the rim of the channel is believed to account, at least in part, for some observed differences in HDAC selectivity between pan-HDAC inhibitors, such as SAHA and selective HDAC inhibitors such as the depsipeptides.

Alkylcarbamoyl naphthalenyloxy octenoyl hydroxyamide is an HDAC inhibitor undergoing clinical investigation in multiple types of solid tumors and hematological cancers. Alkylcarbamoyl naphthalenyloxy octenoyl hydroxyamide is rapidly absorbed and has a half-life of about 7 to 8 hours and, importantly, changes in histone acetylation persists for several days following the administration.

Programmed Cell Death-1 (PD-1) is a cell surface receptor that is a member of the CD28 family of T-cell regulators, within the immunoglobulin superfamily of receptors. The human PD-1 gene is located at chromosome 2q37, and the full-length PD-1 cDNA encodes a protein with 288 amino acid residues with 60% homology to murine PD-1. It is present on CD4- CD8-(double negative) thymocytes during thymic development and is expressed upon activation in mature hematopoietic cells such as T and B cells, NKT cells and monocytes after prolonged antigen exposure. PD-L1 has recently been shown to be expressed on a number of mouse and human tumors (and is inducible by IFN gamma on the majority of PD-L1 negative tumor cell lines) and is postulated to mediate immune evasion (Iwai Y. et al., Proc. Natl. Acad. Sci. U.S.A. 99: 12293-12297 (2002); Strome S. E. et al., Cancer Res., 63: 6501-6505 (2003). In humans, expression of PD-1 and/or PD-L1 has been found in a number of primary tumor biopsies from cancers of the lung, liver, ovary, cervix, skin, colon, glioma, bladder, breast, kidney, esophagus, stomach, oral squamous cell, urothelial cell, and pancreas as well as tumors of the head and neck (Brown J. A. et al., J. Immunol. 170: 1257-1266 (2003); Dong H. et al., Nat. Med. 8: 793-800 (2002); Wintterle et al., Cancer Res. 63: 7462-7467 (2003); Strome S. E. et al., Cancer Res., 63: 6501-6505 (2003); Thompson R. H. et al., Cancer Res. 66: 3381-5 (2006); Thompson et al., Clin. Cancer Res. 13: 1757-61 (2007); Nomi T. et al., Clin. Cancer Res. 13: 2151-7. (2007)). PD-ligand expression on tumor cells has been correlated to poor prognosis of cancer patients across multiple tumor types (reviewed in Okazaki and Honjo, Int. Immunol. 19: 813-824 (2007)).

In addition, it is known that the high expression of PD-L1 on tumor cells is correlated with poor prognosis and survival in various other solid tumor types. It is contemplated that the PD-1/PD-L1 pathway plays a critical role in the tumor immune evasion and could be considered an attractive target for therapeutic intervention in several solid organ types. Several PD-1 and PD-L1 antibodies are in clinical development and, overall, they have been reported to be well tolerated, with most not reaching dose-limiting toxicity in their phase I studies. Several studies have shown that interaction of PD-1 with its ligands (PD-L1 and PD-L2) leads to the inhibition of lymphocyte proliferation in vitro and in vivo. Accordingly, it is contemplated that binding of the ligand PD-L1 to PD-1 downregulates effector anti-tumor T-cell activity and facilitates immune evasion.

Moreover, the disruption of the PD-1/PD-L1 interaction has been shown to increase T cell proliferation and cytokine production and block progression of the cell cycle. In vitro studies of PD-1 blockade by PD-1-specific antibody showed augmentation of cytotoxic T cell responses to liver cancer specific antigens including the increased frequencies of IFN-γ-secreting antigen-specific cells. However, it is contemplated that targeting PD-1 may act as an effective therapeutic strategy for cancer.

The principal method for targeting PD-1 has been through the development of genetically engineered monoclonal antibodies that inhibit functions by interfering with the binding of PD-1 and PD-L1. The anti-PD-1 antibody and antigen-binding portion thereof, bind to PD-1 with high specificity and affinity, block the binding of PD-L1 and/or PD-L2, and inhibit the immunosuppressive effect of the PD-1 signaling pathway. In some embodiments, the combination therapy comprises administering alkylcarbamoyl naphthalenyloxy octenoyl hydroxyamide, a derivative thereof or a salt thereof and an anti-PD-1 antibody or antigen-binding portion thereof, wherein the antibody or antigen-binding portion thereof is a chimeric, humanized or human monoclonal antibody or a portion thereof. In certain embodiments, the anti-PD-1 antibody or antigen-binding portion thereof is a humanized antibody. In some embodiments, the anti-PD-1 antibody or antigen-binding portion thereof is a human antibody. In some embodiments, the anti-PD-1 antibody or antigen-binding portion thereof is a monoclonal antibody or antigen-binding portion thereof.

According to one embodiment, the HDAC inhibitor may include alkylcarbamoyl naphthalenyloxy octenoyl hydroxyamide, a derivative thereof, or a salt thereof. The alkylcarbamoyl naphthalenyloxy octenoyl hydroxyamide is represented by the formula 1: 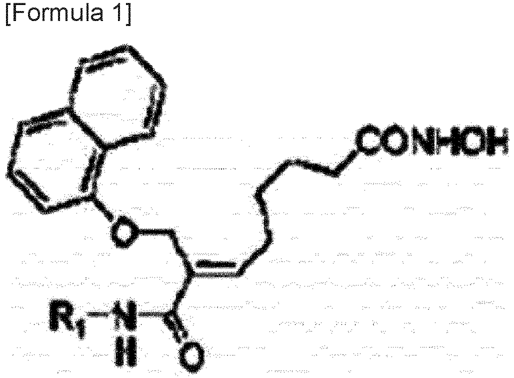 wherein, R₁ is C₁₋₃ alkyl which is unsubstituted or substituted by substituents; pyrrolidine which is unsubstituted or substituted by C₃₋₈ cycloalkyl, C₃₋₈ cycloalkyl C₁₋₃ alkyl, benzyl, C₁₋₃ alkyl or C₃₋₈ cycloalkylcarbonyl; piperidine substituted with C₁₋₃ alkyl or C₃₋₈ cycloalkyl; furan; or C₃₋₈ cycloalkyl,
with the proviso that unsubstituted C₁₋₂ alkyl and C₁₋₂ alkyl substituted with C₁₋₂ alkylpyrrolidinyl are excluded,
wherein the salt may be selected from a phosphoric acid salt, a tartaric acid salt, a stearic acid salt, a gluconic acid salt, a fumaric acid salt, a naphthoic acid salt, a 1-hydroxy-2 salt and a mixture thereof, for example a phosphoric acid salt.

According to one embodiment, the salt may be selected from a phosphoric acid salt, a tartaric acid salt and a mixture thereof, which have relatively high stability and water solubility, for example it may comprise a phosphoric acid salt.

The preferred compounds as the alkylcarbamoyl naphthalenyloxy octenoyl hydroxyamide of formula 1 or a derivative thereof may be selected from the group consisting of following compounds:
1) (E)-N1-(3-(1H-imidazol-1-yl)propyl)-N8-hydroxy-2-((naphthalen-1-yloxy)methyl)octenediam ide,
2) (E)-N8-hydroxy-N1-(4-hydroxyphenethyl)-2-((naphthalen-1-yloxy)methyl)-2-octenediamide,
3) (E)-N1-(3-(dimethylamino)-2,2-dimethylpropyl)-N8-hydroxy-2-((naphthalen-1-yloxy)methyl)octenediamide,
4) (E)-N1-(2-(diisopropylamino)ethyl)-N8-hydroxy-2-((naphthalen-1-yloxy)methyl)octenediam ide,
5) (E)-N8-hydroxy-N1-(1-methoxypropan-2-yl)-2-((naphthalen-1-yloxy)methyl)-2-octenediamide,
6) (E)-N8-hydroxy-N1-(4-methoxybenzyl)-2-((naphthalen-1-yloxy)methyl)-2-octenediamide,
7) (E)-N1-(4-fluorophenethyl)-N8-hydroxy-2-((naphthalen-1-yloxy)methyl)-2-octenediamide,
8) (E)-N8-hydroxy-2-((naphthalen-1-yloxy)methyl)-N1-(tetrahydrofuran-2-yl)methyl)-2-octenediamide,
9) (E)-N1-(2-cyclohexenylethyl)-N8-hydroxy-2-((naphthalen-1-yloxy)methyl)-2-octenediamide,
10) (E)-N8-hydroxy-2-((naphthalen-1-yloxy)methyl)-N1-(3-(2-oxopyrrolidin-1-yl)propyl)-2-octenediamide,
11) (E)-N1-(furan-2-ylmethyl)-N8-hydroxy-2-((naphthalen-1-yloxy)methyl)-2-octenediamide,
12) (E)-N1-(4-(dimethylamino)benzyl)-N8-hydroxy-2-((naphthalen-1-yloxy)methyl)-2-octenediamide,
13) (E)-N8-hydroxy-N1-(2-methoxyethyl)-2-((naphthalen-1-yloxy)methyl)-2-octenediamide,
14) (E)-N1-cyclohexyl-N8-hydroxy-2-((naphthalen-1-yloxy)methyl)-2-octenediamide,
15) (E)-N8-hydroxy-2-((naphthalen-1-yloxy)methyl)-N1-(thiophen-2-ylmethyl)-2-octenediamide,
16) (E)-N8-hydroxy-N1-(4-methoxyphenethyl)-2-((naphthalen-1-yloxy)methyl)-2-octenediamide,
17) (E)-N8-hydroxy-2-((naphthalen-1-yloxy)methyl)-N1-(4-(trifluoromethoxy)benzyl)-2-octenediamide,
18) (E)-N1-(1-(cyclohexylmethyl)pyrrolidin-3-yl)-N8-hydroxy-2-((naphthalen-1-yloxy)methyl)-2-octenediamide,
19) (E)-N1-(1-cyclopentylpiperidin-4-yl)-N8-hydroxy-2-((naphthalen-1-yloxy)methyl)-2-octenediamide,
20) (E)-N1-(1-benzylpyrrolidin-3-yl)-N8-hydroxy-2-((naphthalen-1-yloxy)methyl)-2-octenediamide,
21) (E)-N8-hydroxy-N1-(1-isopropylpyrrolidin-3-yl)-2-((naphthalen-1-yloxy)methyl)-2-octenediamide,
22) (E)-N1-(1-(cyclohexanecarbonyl)pyrrolidin-3-yl)-N8-hydroxy-2-((naphthalen-1-yloxy)methyl)-2-octenediamide,
23) (E)-3-(8-(hydroxyamino)-2-((naphthalen-1-yloxy)methyl)-8-oxo-2-octenamido)pyrrolidine-1-carboxylic acid t-butyl ester,
24) (E)-N8-hydroxy-2-((naphthalen-1-yloxy)methyl)-N1-(pyrrolidin-3-yl)-2-octenediamide,
25) (E)-N1-(1-cyclohexylpyrrolidin-3-yl)-N8-hydroxy-2-((naphthalen-2-yloxy)methyl)-2-octenediamide,
26) (E)-N1-(1-cyclopropylpyrrolidin-3-yl)-N8-hydroxy-2-((naphthalen-1-yloxy)methyl)-2-octenediamide,
27) (E)-N1-(1-cyclopropylpiperidin-4-yl)-N8-hydroxy-2-((naphthalen-1-yloxy)methyl)-2-octenediamide,
28) (E)-N1-(1-ethylpiperidin-4-yl)-N8-hydroxy-2-((naphthalen-1-yloxy)methyl)-2-octenediamide,
29) (E)-N1-(1-ethylpyrrolidin-3-yl)-N8-hydroxy-2-((naphthalen-1-yloxy)methyl)-2-octenediamide,
30) (E)-N8-hydroxy-N1-(2-(1-methylpyrrolidin-2-yl)ethyl)-2-((naphthalen-1-yloxy)methyl)-2-octenediamide,
31) (E)-N8-hydroxy-N1-(1-isopropylpiperidin-4-yl)-2-((naphthalen-1-yloxy)methyl)-2-octenediamide, and
32) (E)-N1-(3-(dimethylamino)propyl)-N8-hydroxy-2-((naphthalen-1-yloxy)methyl)-2-octenediamide.

According to one embodiment, the HADC inhibitor included within the present invention may comprise a phosphoric acid salt of (E)-N1-(3-(dimethylamino)propyl)-N8-hydroxy-2-((naphthalen-1-yloxy)methyl)oct-2-enediamide), which has the structure formula as shown in formula 2.

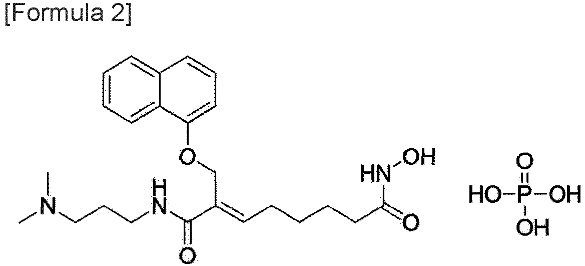

According to one embodiment, the anti-PD-1 antibody of the present invention may comprise one or more selected from the group consisting of pembrolizumab, nivolumab, camrelizumab, cemiplimab, sintilimab and toripalimab.

According to one embodiment, the anti-PD-1 antibody of the present invention can be replaced with an anti-PD-L1 antibody, and for example, the anti-PD-L1 antibody may comprise one or more selected from the group consisting of atezolizumab, avelumab and durvalumab.

According to another embodiment, the anti-PD-L1 may be replaced with an anti-CTLA4 antibody, an anti-VEGFR antibody or an anti-VEGF antibody.

According to one embodiment, the weight ratio of the alkylcarbamoyl naphthalenyloxy octenoyl hydroxyamide, a derivative thereof or a salt thereof to the anti-PD-1 antibody or the anti-PD-L1 antibody may be 1: 0.1 to 1: 15, such as 1: 1 to 1: 5.

According to one embodiment, the alkylcarbamoyl naphthalenyloxy octenoyl hydroxyamide, a derivative thereof or a salt thereof may be administered at a dose of 10 to 500 mg/kg, such as 40 to 250 mg/kg, such as 5 to 50 mg/kg.

According to one embodiment, the anti-PD-1 antibody or the anti-PD-L1 antibody may be administered at a dose of 1 to 200 mg/kg, such as 1 to 10 mg/kg. In another embodiment, the anti-PD-1 antibody or the anti-PD-L1 antibody may be administered at a fixed dose of 200 mg, regardless of the weight of the subject to be administered.

According to one embodiment, the cancer cells of interest in the present invention may be characterized by the expression of PD-L1, and can be screened by measuring expression of PD-L1 in tumor tissues. For example, cancer cells may be characterized by overexpression or low expression of PD-L1. In addition, for example, those having PD-L1 tumor proportion score (TPS) on cancer cells of 1% or more, such as 20% or more, such as 50% or more can be selected as the cancer cells of interest.

The cancer cells may include, specifically, liver cancer cells by hepatitis A virus, liver cancer cells by hepatitis B virus, liver cancer cells by hepatitis C virus, non-viral-related liver cancer cells, metastatic liver cancer cells, colon cancer cells, pancreatic cancer cells, blood cancer cells, melanoma cells or lung cancer cells. For example, the melanoma may be unresectable or metastatic melanoma and the lung cancer may be non-small cell lung cancer, the non-small cell lung cancer being selected from adenocarcinoma, squamous cell carcinoma, non-squamous cell carcinoma and large cell carcinoma.

In addition, the cancer cells may or may not respond to growth degradation or death when treated with existing drugs including sorafenib, lenvatinib, regorafenib, nivolumab or pembrolizumab, and may have been subjected to prior therapy using the above substances.

According to one embodiment, cancer cells of interest in the present invention may or may not show progress by prior therapy using an anti-CTLA4 antibody or a BRAF inhibitor in addition to substances as described above.

According to one embodiment, the treatment sequence of the alkylcarbamoyl naphthalenyloxy octenoyl hydroxyamide or a salt thereof and the anti-PD-1 antibody or the anti-PD-L1 antibody is not particularly limited, and may be in any order, simultaneously or sequentially. Thus, the alkylcarbamoyl naphthalenyloxy octenoyl hydroxyamide, a derivative there of or a salt thereof, and the anti-PD-1 antibody or the anti-PD-L1 antibody may be administered simultaneously, sequentially or in reverse sequence, and specifically they may be administered simultaneously as a single preparation, or may be administered simultaneously or sequentially or in reverse sequence as a separate preparation.

According to one embodiment, the alkylcarbamoyl naphthalenyloxy octenoyl hydroxyamide, a derivative there of or a salt thereof, and the anti-PD-1 antibody or the anti-PD-L1 antibody may be administered simultaneously as they are prepared as a single preparation.

In addition, the alkylcarbamoyl naphthalenyloxy octenoyl hydroxyamide, a derivative there of or a salt thereof, and the anti-PD-1 antibody or the anti-PD-L1 antibody may be prepared in a separate composition, respectively.

According to one embodiment, the alkylcarbamoyl naphthalenyloxy octenoyl hydroxyamide, a derivative thereof or a salt thereof, and the anti-PD-1 antibody or the anti-PD-L1 antibody may be administered at a dose less than or equal to a therapeutically effective dose, respectively. For example, when administered in combination with other types of anticancer therapeutics, the dosage may be adjusted and used as an adjuvant.

According to one embodiment, when administered in combination with other therapeutic agents, 1) the alkylcarbamoyl naphthalenyloxy octenoyl hydroxyamide, a derivative thereof or a salt thereof is administered prior to administration of the anti-PD-1 antibody or the anti-PD-L1 antibody, or
2) the anti-PD-1 antibody or the anti-PD-L1 antibody may be administered prior to administration of the alkylcarbamoyl naphthalenyloxy octenoyl hydroxyamide, a derivative thereof or a salt thereof.

According to one embodiment, cancer cell death can be induced by treating animals with the two substances for 1 to 40 days, for example, 3 to 21 days. Specifically, the alkylcarbamoyl naphthalenyloxy octenoyl hydroxyamide, a derivative thereof or a salt thereof may be repeatedly administered for 3 weeks with, for example, 1 treatment cycle consisting of 5 consecutive days of drug administration, followed by 2 consecutive days of non-administration. In addition, the anti-PD-1 antibody or the anti-PD-L1 antibody may be administered on the first day of the treatment cycle, and may be administered every other week.

The method of administration may be selected from a number of techniques of administering a compound existing in the art including, but not limited to, oral, injection, aerosol, parenteral, and topical administration. Pharmaceutical compositions can also be obtained by reacting the compound with an inorganic or organic acid, such as hydrochloric acid, hydrobromic acid, sulfuric acid, nitric acid, phosphoric acid, methanesulfonic acid, ethanesulfonic acid, p-toluenesulfonic acid, salicylic acid and the like.

The treatment method is not particularly limited, but according to one embodiment, the alkylcarbamoyl naphthalenyloxy octenoyl hydroxyamide, a derivative thereof or a phosphoric acid salt thereof may be administered intravenously or orally to an animal, and the anti-PD1 antibody or anti-PD-L1 antibody may be administered intravenously to the animal.

The pharmaceutic composition of the present invention may be provided in the form of tablets, granules, powders, capsules, dry syrups or injections. Specifically, it may be provided in any convenient form, such as in the form of tablets, pellets, granules, capsules, suspensions, emulsions or powders which are suitable for reconstituted with water or other suitable liquid medium. In addition, it may be provided in the form of oral dosage forms or injections.

The pharmaceutical composition for oral administration may comprise one or more diluents selected from the group consisting of microcrystalline cellulose, mannitol, lactose and lactose, one or more lubricants selected from the group consisting of talc, magnesium stearate, sodium stearyl fumarate and glyceryl behenate, and one or more binders selected from the group constisting of polyvinylpyrrolidone, hydroxypropylmethylcellulose and hydroxypropylcellulose. In addition, for example, the coating layer may be included in an amount of 1 to 10% by weight based on 100 parts by weight of the tablet or capsule. Specifically, for example, the coating layer may include a water-soluble coating base, and as the coating base a commonly used coating base may be used. More specifically, for example, it may include a coating base containing polyvinyl alcohol derivatives, methacrylic acid derivatives, and polyacrylic acid derivatives, and for example, one or two or more selected from the group consisting of Opadry^{®}, Kollicoat^{®}, and hydroxypropyl methyl cellulose (HPMC) may be used, such as polyvinyl alcohol-containing Opadry^{®} having relatively excellent moisture and light blocking effects.

When preparing a granular composition for oral administration, it is preferable not to use purified water as a binding solvent in consideration of unstable moisture stability characteristics, but ethanol, which is easily removed during the manufacturing process, can be used. An alternative for magnesium stearate which is a conventional lubricant used in a composition for oral administration may be used as the magnesium stearate may not be compatible with the alkylcarbamoyl naphthalenyloxy octenoyl hydroxyamide of the present invention.

According to one embodiment, in addition to the above-described additives, a pharmaceutically acceptable excipient having excellent compatibility with the compound may be added.

The composition for injection may be provided in the liquid form because the alkylcarbamoyl naphthalenyloxy octenoyl hydroxyamide is a water-soluble substance with high solubility in water. The composition generally does not require the use of solubilizers and other additives used for poorly soluble substances. It is preferable to use additives minimally because compatibility with additives can be unstable. More specifically, the composition may be prepared by dissolving in nitrogen-purged water for injection, and then freezing-drying.

According to one embodiment, the method may further comprise the step of sterilizing. Sterilization treatment methods may include dry heat sterilization, high-pressure or reduced pressure sterilization, filter sterilization, gas sterilization, radiation sterilization, and the like. The filtration sterilization may use, for example, a nitrocellulose membrane filter, for example, a 0.45 µm filter or a 0.2 µm filter, etc. In the present invention, the method may further comprise, for example, the step of sterilizing by high temperature and reduced pressure sterilization or aseptic filtration.

According to another embodiment of the present invention, there can be provided a pharmaceutical composition for an anticancer agent comprising alkylcarbamoyl naphthalenyloxy octenoyl hydroxyamide, a derivative thereof or a salt thereof, and an anti-PD-1 antibody or an anti-PD-L1 antibody.

Hereinafter, embodiments of the present invention will be described in detail so that those of ordinary skill in the art can easily carry out the present invention. However, the present invention may be embodied in various different forms and is not limited to the embodiments described herein.

### Experimental Example 1: Tumor growth inhibition rate in mouse model

As shown in Table 1, in 9 mouse models for liver cancer in which Hepa1-6 hepatoma cells were transplanted into mice (C57BL/6), it was confirmed that the tumor growth inhibitory ability was improved when treated with alkylcarbamoyl naphthalenyloxy octenoyl hydroxyamide (HDAC inhibitor) or anti-PD-1 antibodies and it was confirmed that the tumor disappeared when treated with a combination of two substances.

**[Table 1]**

| Example 1 | HDAC inhibitor and anti-PD-1 antibody |
|---|---|
| Comparative Example 1 | HDAC inhibitor |
| Comparative Example 2 | anti-PD-1 antibody |

(E)-N1-(3-(dimethylamino)propyl)-N8-hydroxy-2-((naphthalen-1-yloxy)methyl)-2-octene diamide phosphate (CG-745, CAS No. 2173017-02-0) was used as the alkylcarbamoyl naphthalenyloxy octenoyl hydroxyamide compound, and BP0146 (BioXcell, West Lebanon, NH, USA) was used as the anti-PD-1 antibody.

1 x 10⁶ Hepa1-6 hepatoma cells were injected into the left flank of 6-week-old C57BL6 mice. After 5 days, they were separated into 4 groups for administration of the vehicle, CG-745 alone, the anti-PD-1 antibody alone, or a combination of CG-745 and the anti-PD-1 antibody. The tumor volume was defined as (long axis) x (short axis)² x 0.5. By measuring the tumor volume, mice with the tumor volume smaller than 1 mm were considered tumor-free and they were observed for at least for 50 days. Vehicle, CG-745, and the anti-PD-1 antibody were administered intraperitoneally (I.P). CG-745 was administered at a dose of 20 mg/kg for 5 consecutive days followed by 2 days of rest (1 treatment cycle) and the treatment cycle was repeated 3 times. The anti-PD-1 antibody was administered once at a dose of 5 mg/kg together with CG-745 on the fifth day of the first treatment cycle of CG-745.

The average inhibition rate of tumor growth for 9 mice is shown in Fig. 1, and the results for each of 9 mice are shown in Fig. 2.

As shown in the graph of Fig. 1, it was found that on Day 32, Comparative Example 1 showed a tumor growth inhibition rate of 73.8% and Comparative Example 2 showed 84.59%, respectively. In addition, it was found that the tumor disappeared in 4 mice when treated with Comparative Example 1. In particular, it was found that on Day 32, Example 1 showed a tumor growth inhibition rate of 111.18% and the tumor disappeared in all 9 mice. Therefore, it was confirmed that the direct or indirect anticancer activity by the alkylcarbamoyl naphthalenyloxy octenoyl hydroxyamide was increased by the anti-PD-1 antibody.

As described above, the present invention confirmed that the combination of alkylcarbamoyl naphthalenyloxy octenoyl hydroxyamide as an HDAC inhibitor and an anti-PD-1 antibody has an effective effect on the killing of cancer cells.

The above descriptions are merely illustrative of the technical idea of the present invention, and those of ordinary skill in the technical field to which the present invention pertains can make various modifications and variations without departing from the essential characteristics of the present invention. In addition, the embodiments disclosed in the present invention are not intended to limit the technical idea of the present invention, but to explain the technical idea, and the scope of the technical idea of the present invention is not limited by these embodiments. The scope of protection of the present invention should be interpreted by the appended claims, and all technical ideas within the scope equivalent thereto should be interpreted as being included in the scope of the present invention.

## Claims

1. A pharmaceutical composition for treating or preventing cancer comprising:
alkylcarbamoyl naphthalenyloxy octenoyl hydroxyamide, a derivative thereof or a salt thereof; and
an anti-PD-1 antibody or an anti-PD-L1 antibody.

2. The pharmaceutical composition according to claim 1, wherein the weight ratio of the alkylcarbamoyl naphthalenyloxy octenoyl hydroxyamide, a derivative thereof or a salt thereof to the anti-PD-1 antibody or the anti-PD-L1 antibody is 1:0.1 to 1:15.

3. The pharmaceutical composition according to claim 1, wherein the dose of the anti-PD-1 antibody or the anti-PD-L1 antibody is 1 to 200 mg/kg.

4. The pharmaceutical composition according to claim 1, wherein the dose of the alkylcarbamoyl naphthalenyloxy octenoyl hydroxyamide, a derivative thereof or a salt thereof is 10 to 500 mg/kg.

5. The pharmaceutical composition according to claim 1, wherein the anti-PD-1 antibody comprises one or more selected from the group consisting of pembrolizumab, nivolumab, camrelizumab, cemiplimab, sintilimab and toripalimab.

6. The pharmaceutical composition according to claim 1, wherein the anti-PD-L1 antibody comprises one or more selected from the group consisting of atezolizumab, avelumab and durvalumab.

7. The pharmaceutical composition according to claim 1, wherein the alkylcarbamoyl naphthalenyloxy octenoyl hydroxyamide or a derivative thereof is one or more selected from the group consisting of following compounds:
1) (E)-N1-(3-(1H-imidazol-1-yl)propyl)-N8-hydroxy-2-((naphthalen-1-yloxy)methyl)octenediam ide,
2) (E)-N8-hydroxy-N1-(4-hydroxyphenethyl)-2-((naphthalen-1-yloxy)methyl)-2-octenediamide,
3) (E)-N1-(3-(dimethylamino)-2,2-dimethylpropyl)-N8-hydroxy-2-((naphthalen-1-yloxy)methyl)octenediamide,
4) (E)-N1-(2-(diisopropylamino)ethyl)-N8-hydroxy-2-((naphthalen-1-yloxy)methyl)octenediam ide,
5) (E)-N8-hydroxy-N1-(1-methoxypropan-2-yl)-2-((naphthalen-1-yloxy)methyl)-2-octenediamide,
6) (E)-N8-hydroxy-N1-(4-methoxybenzyl)-2-((naphthalen-1-yloxy)methyl)-2-octenediamide,
7) (E)-N1-(4-fluorophenethyl)-N8-hydroxy-2-((naphthalen-1-yloxy)methyl)-2-octenediamide,
8) (E)-N8-hydroxy-2-((naphthalen-1-yloxy)methyl)-N1-(tetrahydrofuran-2-yl)methyl)-2-octenediamide,
9) (E)-N1-(2-cyclohexenylethyl)-N8-hydroxy-2-((naphthalen-1-yloxy)methyl)-2-octenediamide,
10) (E)-N8-hydroxy-2-((naphthalen-1-yloxy)methyl)-N1-(3-(2-oxopyrrolidin-1-yl)propyl)-2-octenediamide,
11) (E)-N1-(furan-2-ylmethyl)-N8-hydroxy-2-((naphthalen-1-yloxy)methyl)-2-octenediamide,
12) (E)-N1-(4-(dimethylamino)benzyl)-N8-hydroxy-2-((naphthalen-1-yloxy)methyl)-2-octenediamide,
13) (E)-N8-hydroxy-N1-(2-methoxyethyl)-2-((naphthalen-1-yloxy)methyl)-2-octenediamide,
14) (E)-N1-cyclohexyl-N8-hydroxy-2-((naphthalen-1-yloxy)methyl)-2-octenediamide,
15) (E)-N8-hydroxy-2-((naphthalen-1-yloxy)methyl)-N1-(thiophen-2-ylmethyl)-2-octenediamide,
16) (E)-N8-hydroxy-N1-(4-methoxyphenethyl)-2-((naphthalen-1-yloxy)methyl)-2-octenediamide,
17) (E)-N8-hydroxy-2-((naphthalen-1-yloxy)methyl)-N1-(4-(trifluoromethoxy)benzyl)-2-octenediamide,
18) (E)-N1-(1-(cyclohexylmethyl)pyrrolidin-3-yl)-N8-hydroxy-2-((naphthalen-1-yloxy)methyl)-2-octenediamide,
19) (E)-N1-(1-cyclopentylpiperidin-4-yl)-N8-hydroxy-2-((naphthalen-1-yloxy)methyl)-2-octenediamide,
20) (E)-N1-(1-benzylpyrrolidin-3-yl)-N8-hydroxy-2-((naphthalen-1-yloxy)methyl)-2-octenediamide,
21) (E)-N8-hydroxy-N1-(1-isopropylpyrrolidin-3-yl)-2-((naphthalen-1-yloxy)methyl)-2-octenediamide,
22) (E)-N1-(1-(cyclohexanecarbonyl)pyrrolidin-3-yl)-N8-hydroxy-2-((naphthalen-1-yloxy)methyl)-2-octenediamide,
23) (E)-3-(8-(hydroxyamino)-2-((naphthalen-1-yloxy)methyl)-8-oxo-2-octenamido)pyrrolidine-1-carboxylic acid t-butyl ester,
24) (E)-N8-hydroxy-2-((naphthalen-1-yloxy)methyl)-N1-(pyrrolidin-3-yl)-2-octenediamide,
25) (E)-N1-(1-cyclohexylpyrrolidin-3-yl)-N8-hydroxy-2-((naphthalen-2-yloxy)methyl)-2-octenediamide,
26) (E)-N1-(1-cyclopropylpyrrolidin-3-yl)-N8-hydroxy-2-((naphthalen-1-yloxy)methyl)-2-octenediamide,
27) (E)-N1-(1-cyclopropylpiperidin-4-yl)-N8-hydroxy-2-((naphthalen-1-yloxy)methyl)-2-octenediamide,
28) (E)-N1-(1-ethylpiperidin-4-yl)-N8-hydroxy-2-((naphthalen-1-yloxy)methyl)-2-octenediamide,
29) (E)-N1-(1-ethylpyrrolidin-3-yl)-N8-hydroxy-2-((naphthalen-1-yloxy)methyl)-2-octenediamide,
30) (E)-N8-hydroxy-N1-(2-(1-methylpyrrolidin-2-yl)ethyl)-2-((naphthalen-1-yloxy)methyl)-2-octenediamide,
31) (E)-N8-hydroxy-N1-(1-isopropylpiperidin-4-yl)-2-((naphthalen-1-yloxy)methyl)-2-octenediamide, and
32) (E)-N1-(3-(dimethylamino)propyl)-N8-hydroxy-2-((naphthalen-1-yloxy)methyl)-2-octenediamide.

8. The pharmaceutical composition according to claim 1, wherein the salt of alkylcarbamoyl naphthalenyloxy octenoyl hydroxyamide is (E)-N1-(3-(dimethylamino)propyl)-N8-hydroxy-2-((naphthalene-1-yloxy)methyl)-2-octenediamide phosphate.

9. The pharmaceutical composition according to claim 1, wherein:
the alkylcarbamoyl naphthalenyloxy octenoyl hydroxyamide, a derivative there of or a salt thereof; and
the anti-PD-1 antibody or the anti-PD-L1 antibody are administered simultaneously as a single preparation, or are administered simultaneously or sequentially or in reverse sequence as a separate preparation.

10. The pharmaceutical composition according to claim 1, wherein:
the alkylcarbamoyl naphthalenyloxy octenoyl hydroxyamide, a derivative thereof or a salt thereof; and
the anti-PD-1 antibody or the anti-PD-L1 antibody are prepared as a single preparation.

11. The pharmaceutical composition according to claim 1, wherein:
the alkylcarbamoyl naphthalenyloxy octenoyl hydroxyamide, a derivative thereof or a salt thereof; and
the anti-PD-1 antibody or the anti-PD-L1 antibody are prepared in a separate composition, respectively.

12. The pharmaceutical composition according to claim 1, wherein:
the alkylcarbamoyl naphthalenyloxy octenoyl hydroxyamide, a derivative thereof or a salt thereof; and
the anti-PD-1 antibody or the anti-PD-L1 antibody are administered at a dose less than or equal to a therapeutically effective dose, respectively.

13. The pharmaceutical composition according to claim 1, wherein:
1) the alkylcarbamoyl naphthalenyloxy octenoyl hydroxyamide, a derivative thereof or a salt thereof is administered prior to administration of the anti-PD-1 antibody or the anti-PD-L1 antibody, or
2) the anti-PD-1 antibody or the anti-PD-L1 antibody is administered prior to administration of the alkylcarbamoyl naphthalenyloxy octenoyl hydroxyamide, a derivative thereof or a salt thereof.

14. The pharmaceutical composition according to claim 1, wherein the alkylcarbamoyl naphthalenyloxy octenoyl hydroxyamide, a derivative thereof or a salt thereof, and the anti-PD-1 antibody or the anti-PD-L1 antibody are formulated as a separate preparation such that the alkylcarbamoyl naphthalenyloxy octenoyl hydroxyamide, a derivative thereof or a salt thereof is administered intravenously or orally to an animal and the anti-PD-1 antibody or the anti-PD-L1 antibody is administered intravenously to the animal.

15. The pharmaceutical composition according to claim 1, wherein the composition is synergistic in inhibiting the growth of or killing cancer cells.

16. The pharmaceutical composition according to claim 15, wherein the cancer cells are **characterized by** the expression of PD-L1.

17. The pharmaceutical composition according to claim 15, wherein the cancer cells are liver cancer cells by hepatitis A virus, liver cancer cells by hepatitis B virus, liver cancer cells by hepatitis C virus, non-viral-related liver cancer cells, metastatic liver cancer cells, colon cancer cells, pancreatic cancer cells, blood cancer cells, melanoma cells or lung cancer cells.

18. The pharmaceutical composition according to claim 15, wherein the cancer cells are screened by a method comprising measuring the expression of PD-L1 in the tumor tissue.

19. The pharmaceutical composition according to claim 15, wherein PD-L1 tumor proportion score (TPS) on the cancer cells is 1 % or more.
